# EUROPÄISCHE PATENTSCHRIFT

(11) **EP 3 831 350 B1**
(45) Veröffentlichungstag und Bekanntmachung des Hinweises auf die Patenterteilung: **23.11.2022**
(21) Anmeldenummer: 20206494.5
(22) Anmeldetag: 12.08.2014
(51) Int. Cl.: A61F 13/20, A61F 13/00, A61F 13/02, A61F 13/06, A61F 13/10, A61F 13/14

(54) **WUNDPFLEGEARTIKEL AUFWEISEND EINE IM WESENTLICHEN POLYGONALE ODER ELLIPOSIDE GRUNDFLÄCHE SOWIE MINDESTENS EINE AN EINER SEITE ANGEORDNETE AUSNEHMUNG**
WOUND CARE ARTICLE HAVING A SUBSTANTIALLY POLYGONAL OR ELLIPSOID MAIN SURFACE AND AT LEAST ONE RECESS ARRANGED ON ONE SIDE
ARTICLE POUR LE SOIN DES PLAIES PRÉSENTANT UNE SURFACE DE BASE SENSIBLEMENT POLYGONALE OU ELLIPSOÏDALE ET AU MOINS UN ÉVIDEMENT PLACÉ SUR UN CÔTÉ

(30) Priorität: 12.08.2013 DE 202013103639 U; 02.09.2013 DE 202013103953 U
(43) Veröffentlichungstag der Anmeldung: 09.06.2021
(62) Teilanmeldung aus: 14752300.5
(73) Patentinhaber: BSN medical GmbH, 22761 Hamburg (DE)
(72) Erfinder: Riesinger, Birgit, 48149 Münster (DE)
(74) Vertreter: Cohausz & Florack

(56) Entgegenhaltungen:
- WO-A1-92/05756
- DE-U1- 20 207 356
- DE-U1-202012 101 743
- US-A- 2 875 758
- US-A1- 2004 049 146
- US-A1- 2008 294 136

## Beschreibung

Wundpflegeartikel gemäß dem Oberbegriff des Anspruchs 1 sind beispielsweise aus der EP 1507498 bekannt und haben sich in der Versorgung chronischer und stark exsudierender Wunden sehr bewährt.

Sie weisen eine im Wesentlichen flächige Form auf und werden mittels geeigneter Mittel im Bereich einer Körperwunde angeordnet.

Gerade bei größeren Wundpflegeartikeln kann es vorkommen, dass die betreffende Körperpartie unregelmäßig ist und Vertiefungen, Kurven oder Erhöhungen aufweist. Ein im Wesentlichen flächiger Wundpflegeartikel wird einer solchen Anatomie nicht gerecht, und es kommt zu Faltenwurf oder Knicken, die der Patient als unangenehm empfinden kann, und die auch die Wirksamkeit des Wundpflegeartikels in Mitleidenschaft ziehen können.

Aufgabe der vorliegenden Erfindung ist es, einen Wundpflegeartikel bereitzustellen, der eine gute Anpassbarkeit an die anatomischen Bedingungen verschiedener Körperregionen von Mensch und Tier aufweist.

Diese Aufgabe wird mit den Merkmalen des vorgelegten Hauptanspruchs gelöst.

Gegenstand der Erfindung ist demnach ein flächiger Wundpflegeartikel aufweisend eine im Wesentlichen polygonale oder ellipsoide Grundfläche sowie mindestens eine an einer Seite angeordnete Ausnehmung.

Es hat sich überraschender Weise gezeigt, dass der erfindungsgemäße Wundpflegeartikel durch diese recht einfache Modifikation eine sehr viel höhere Variabilität aufweist und insbesondere eine gute Anpassbarkeit an die anatomischen Bedingungen verschiedener Körperregionen von Mensch und Tier erhält.

Der Begriff "Wundpflegeartikel" soll im Folgenden insbesondere eine Wundauflage, bevorzugt eine flächige Wundauflage oder ein Wundpflegetuch bezeichnen. Besagte Wundauflage enthält eine flächige Lage und kann dabei sowohl absorbierend als auch nicht oder nur unwesentlich absorbierend ausgestaltet sein. Insbesondere kann der Begriff "Wundpflegeartikel" auch als ein Ensemble verschiedener Produkte verstanden werden, die in einer gegebenen Anordnung auf der zu behandelnden Wunde angeordnet werden. Dieses Ensemble kann eine physikalische Einheit bilden, indem die verschiedenen Produkte in einer gemeinsamen Hülle zusammengefasst oder - ggf. ohne Hülle - adhäsiv miteinander verbunden sind. Das Ensemble kann jedoch auch in Form eines Kits vorliegen, bei dem die verschiedenen Produkte mithilfe einer Wickel in der gegebenen Anordnung auf der zu behandelnden Wunde angeordnet sind.

Der Begriff "Ausnehmung" soll eine mehr oder minder konkave Materialentnahme an mindestens einer Seite des Wundpflegeartikels bezeichnen.

Durch besagte Ausnehmung ist gewährleistet, dass der Wundpflegeartikel eine gute Anpassbarkeit an die anatomischen Bedingungen verschiedener Körperregionen von Mensch und Tier aufweist. Die Zeichnungen demonstrieren diesen Vorteil in anschaulicher Art und Weise.

Dabei ist die Grundfläche bevorzugt rechteckig oder von einem Rechteck abgeleitet. Weiterhin ist bevorzugt vorgesehen, dass der Wundpflegeartikel mindestens eine abgerundete oder abgewinkelte Ecke aufweist. Alternativ kann die Grundfläche bevorzugt auch aus einem Trapez abgeleitet sein.

Für den Fall, dass die Grundform des Wundpflegeartikels ein nicht-gleichseitiges Rechteck ist oder aus einem solchen abgeleitet ist, ist bevorzugt vorgesehen, dass die Ausnehmung an einer Längsseite angeordnet ist.

Alternativ ist die Grundfläche bevorzugt kreisförmig, oval oder ellipsoid, oder von einem Kreis, Oval oder einer Ellipse abgeleitet. Dies kann auch bedeuten, dass die Grundfläche einem Halbkreis, einem halben Oval oder einer halben Ellipse entspricht.

Bevorzugt ist vorgesehen, dass die Ausnehmung kreisabschnittsförmig oder ellipsenabschnittsförmig ausgebildet ist. Besonders bevorzugt ist sie halbkreisförmig oder halbellipsenförmig.

Ebenso bevorzugt ist vorgesehen, dass die Ausnehmung rechteckig oder winkelförmig ausgebildet ist. Besonders bevorzugt ist sie V-förmig.

In einer rechteckigen Ausnehmung kann vorgesehen sein, dass das ausgenommene Material als "Lasche" oder "Zunge" weiterhin mit dem Rest der Lage in Verbindung steht.

In all den genannten Fällen hat sich als vorteilhaft herausgestellt, die Ausnehmung nicht lediglich in Form eines oder mehrerer Schlitze auszugestalten. Die erfindungsgemäße Ausnehmung ist breiter angelegt, sodass sie auch ermöglich, dass der ausgenommene Bereich auch von einer etwaig vorhandenen, die flächige Lage umgebenden Hülle ausgenommen wird, und zwar dergestalt, dass letztere die flächige Lage auch im Bereich der Ausnehmung durch eine Naht umgibt. Handelte es sich bei der Ausnehmung um einen Schlitz oder ein sehr schmales Band, könnte diese von der etwaig vorhandenen Hülle nicht ausgenommen werden, da diese stets einen leichten Überstand über die flächige Lage aufwiest, und in diesem Fall nicht genügend Raum für eine Naht auf beiden Seiten der Ausnehmung bestünde.

Zwar sind Wundpflegeartikel bekannt, bei denen die flächige Lage einen oder mehrere Schlitze aufweist, regelmäßig werden diese Schlitze jedoch durch eine Hülle nicht aufgenommen. Solche Schlitze können verschiedene Aufgaben bewerkstelligen, allerdings nicht die erfindungsgemäß vorgesehene bessere anatomische Anpassbarkeit, da die Hülle solche Schlitze in der flächigen Lage nicht ebenfalls ausnehmen kann.

Ferner ist bevorzugt vorgesehen, dass der Wundpflegeartikel mindestens eine flächige Lage aufweisend ein absorbierendes Material aufweist.

Weiterhin ist bevorzugt vorgesehen, dass der Wundpflegeartikel mindestens ein superabsorbierendes Polymer aufweist.

Hierbei ist bevorzugt vorgesehen, dass die flächige Lage mehr als 26; 27; 28; 29; 30; 31; 32; 33; 34; 35; 36; 37; 38; 39; 40; 41; 42; 43; 44; 45; 46; 47; 48; 49; 50; 51; 52; 53; 54; 55; 56; 57; 58; 59; 60; 61; 62; 63; 64; 65; 66; 67; 68; 69; 70; 71; 72; 73; 74; 75; 76; 77; 78; 79; 80; 81; 82; 83; 84; 85; 86; 87; 88; 89; 90; 91; 92; 93; 94; 95; 96; 97; 98 Gewichts-% an Superabsorbierenden Polymeren aufweist.

"Superabsorbierende Polymere" (SAP) sind Kunststoffe, die in der Lage sind, ein Vielfaches ihres Eigengewichts - bis zum 1000-fachen - an Flüssigkeiten aufzusaugen. Chemisch handelt es sich dabei um ein Copolymer aus Acrylsäure (Propensäure, C₃H₄O₂) und Natriumacrylat (Natriumsalz der Acrylsäure, NaC₃H₃O₂), wobei das Verhältnis der beiden Monomere zueinander variieren kann. Zusätzlich wird ein so genannter Kernvernetzer (Core-Cross-Linker, CXL) der Monomerlösung zugesetzt, der die gebildeten langkettigen Polymermoleküle stellenweise untereinander durch chemische Brücken verbindet (sie "vernetzt"). Durch diese Brücken wird das Polymer wasserunlöslich. Beim Eindringen von Wasser oder wässrigen Salzlösungen in den Polymerpartikel quillt er auf und strafft auf molekularer Ebene dieses Netzwerk, so dass das Wasser ohne Hilfe nicht mehr entweichen kann.

Alternativ können die Superabsorber auf Methylacrylsäurebasis, Polyvinylalkohol-Maleinsäureanhydrid-Copolymere, Polysaccharid-Maleinsäureanhydrid-Copolymere, Maleinsäurederivate, Acrylamidopropansulfonsäure-Copolymere, Stärke-Acrylnitril-Pfropfpolymere, gelatinisierte Stärkederivate, Alkyl- oder Hydroxyalkylcellulose, Carboxymethylcellulose, Stärke-Acrylsäure-Pfropfpolymere, Vinylacetat-Acrylsäureester-Copolymere, Acrylnitril- oder Acrylamid-Copolymere gewählt sein.

Die Superabsorbierenden Partikel können in Pulver- oder Granulatform in einer Partikelgröße zwischen 100 und etwa 1000 µm vorliegen.

Ebenso kann es sich bei besagten superabsorbierenden Polymeren auch um Hydrogel-Nanopartikel aufweisend Hydroxy-Terminierte Methacrylatmonomere, wie 2-Hydroxyethylmethacrylat (HEMA) und/oder 2-Hydroxypropylmethacrylat (HPMA), die z.B. als Altrazeal vermarktet werden, handeln.

Dabei weist der Wundpflegeartikel bevorzugt ein Material auf, das ausgewählt ist aus der Gruppe enthaltend eine Matte, insbesondere aus einem Airlaid aufweisend superabsorbierende Polymere, und/oder eine lose Füllung aus superabsorbierenden Polymeren. Besagte Airlaidmatte kann bevorzugt einen im wesentlichen flachen Materialabschnitt aus Absorptionsmaterial aufweisen, der z. B. aus einem aufsaugenden Vlies mit darin verteilten superabsorbierenden Polymeren besteht.

Dieser Wundpflegeartikel kann der absorbierenden Einlage entsprechen, die in einer Wundauflage der Anmelderin der vorliegenden Erfindung enthalten ist, wie sie beispielsweise in der WO03094813, der WO2007051599 und der WO0152780 offenbart ist und unter dem Handelsnamen "sorbion Sachet" vertrieben wird. Der Offenbarungsgehalt der genannten Schriften sei dem Offenbarungsgehalt dieser Schrift vollumfänglich beigefügt.

Der Wundpflegeartikel in einer anderen Ausgestaltung ebenso ein Geflecht von Bändchen aufweisen, die teils aus einem gelbildenden Material und Teils aus einem nicht gelbildenden Material gefertigt sind. Ein solches Geflecht ist beispielsweise aus der EP2153807 bekannt.

Der Wundpflegeartikel kann in einer anderen Ausgestaltung ebenso einen Kern bilden, der - ggf. flockenartige - Fasern oder Garne aus superabsorbierenden Polymeren sowie superabsorbierenden Polymeren in Granulatform aufweist, wobei die Granulate an die Fasern bzw. Garne in mehreren Höhen angeklebt bzw. angeschweißt sind, und die Granulate über mehr als 50 % der gesamten Bauhöhe wenigstens eines Abschnitts des Kerns verteilt sind, wobei vermengte Bereiche von Granulat und Fasern vorliegen. Der Gewichtsanteil der superabsorbierenden Polymere kann dabei bevorzugt im Bereich zwischen 10 - 25 Gew.-% liegen. Ähnliche Konstruktionen sind aus herkömmlichen Inkontinenzmaterialien bekannt und wie Hygienebinden für ihre polsternden Eigenschaften bekannt.

Der Wundpflegeartikel kann in einer anderen Ausgestaltung ein Gemisch aus Superabsorbierenden Fasern, Superabsorbierenden Partikeln, Bicomponentfasern und Zellulosefasern aufweisen.

Der Wundpflegeartikel kann in einer anderen Ausgestaltung ebenso mindestens eine flache Lage, aufweisend Fasern oder Garne aus superabsorbierenden Polymeren enthalten, an welche superabsorbierende Polymere in Granulatform geklebt sind. Dadurch ergibt sich in einer bevorzugten Ausgestaltung ein Aufbau des Körpers, der wenigstens drei Schichten aufweist, wobei zwei Deckschichten eine Schicht aufweisend superabsorbierende Polymere umgeben.

Dabei liegen in der Ebene keine Vermengungen von Fasern und superabsorbierenden Polymeren vor; sondern lediglich fixierte Benachbarungen beider Materialien. Die ggf. vorgesehenen mehreren Lagen können dabei in einer bevorzugten Ausgestaltung auch durch Walzen, Pressen, Kalandrieren oder ähnliche Verfahren physisch miteinander verdichtet sein. Überdies kann der Körper sich wiederholende Musterungen oder Maserungen aufweisen, wie z.B. ein Karomuster, ein Stanzmuster oder dergleichen.

Der Begriff "Vlies" bezeichnet ein textiles Flächengebilde aus einzelnen Fasern, das im Gegensatz zu Geweben, Gestricken und Gewirken nicht aus Garnen hergestellt wird. Vliese behalten ihre strukturelle Integrität i.d.R. durch Haftung der einzelnen Fasern aneinander. Sie werden auch als "Nonwovens" bezeichnet, und z.B. durch Walken der Fasern hergestellt. Der Begriff "Airlaid" bezeichnet einen speziellen Vliesstoff aus Zellstoff und Polyolefinfasern, in den ggf. superabsorbierende Polymere eingebettet sind.

Der Begriff "Exsudat" bezeichnet eine über entzündliche Prozesse vom Blutplasma abgeleitete Wundflüssigkeit. So wie das Blut für den Transport von Nährstoffen und anderen Boten- Stoffen und damit für die Versorgung verschiedener Teile des Körpers verantwortlich ist, dient das Exsudat auf ganz ähnliche Weise der Versorgung des Wundbettes und der darin ablaufenden Heilungsprozesse. Um dieser Vielzahl an Funktionen gerecht zu werden, enthält es ein breites Spektrum an Komponenten, woraus ein spezifisches Gewicht resultiert, das leicht oberhalb dessen von Wasser liegt. Darin unterscheidet es sich auch vom Transsudat, welches von nicht-entzündlichen Prozessen abgeleitet ist und ein deutlich geringeres spezifisches Gewicht mit einem geringen Zell- und Proteingehalt aufweist. Neben der Bereitstellung von Nährstoffen für die Fibroblasten und Epithelzellen beeinflusst die Beschaffenheit des Exsudats die verschiedenen Prozesse der Wundheilung zeitlich und räumlich durch seinen hohen Gehalt an Wachstumsfaktoren und Zytokinen. Diese werden vor allem durch Thrombozyten, Keratinozyten, Makrophagen und Fibroblasten gebildet. Sie beeinflussen die Motilität, Migration und Proliferation der verschiedenen an der Wundheilung beteiligten Zellen. So wird das Einwandern von Zellen in den Wundgrund ebenso gefördert, wie die Versorgung des neugebildeten Granulationsgewebes durch die Angiogenese. Auch die Wundreinigung wird durch das Exsudat unterstützt. Es enthält verschiedene Serin-, Cystein- und Aspartatproteasen sowie Matrix-Metalloproteasen, die in ihrer Aktivität streng reguliert irreversibel geschädigtes Gewebe abbauen und somit das Wundbett für die nachfolgenden Phasen der Heilung vorbereiten. Generell unterscheidet man bei solchen Prozessen zwischen physiologischem und pathologischem Exsudat.

Bestandteile des physiologischen Exsudats sind insbesondere Salze, Glucose, Zytokine und Wachstumsfaktoren, Plasmaproteine, Proteasen (insbesondere Matrix-Metalloproteasen), Granulozyten und Makrophagen.

Weiterhin weist der erfindungsgemäße Wundpflegeartikel starke antimikrobielle Eigenschaften auf, die einerseits durch die Eigenschaft der Superabsorber, Proteine und Bakterien zu binden, bedingt sind, und andererseits auf deren wasserbindende Eigenschaften zurückzuführen sind, durch welche den Bakterien die für die Aktivität notwendige Flüssigkeit entzogen wird.

Weiterhin konnte gezeigt werden, dass die Kombination der verschiedenen superabsorbierenden Materialien eine Modulation von proinflammatorischen Faktoren, wie Matrixmetallo-Proteasen ("MMPs", insbesondere Collagenase und Elastase), Sauerstoffradikale ("ROS"), IL1beta, IL6, IL8 und TNF alpha ermöglicht. Auch dieser Effekt ist auf die bindenden Eigenschaften der superabsorbierenden Polymere gegenüber Proteinen zurückzuführen.

Ferner kommt einem solchen Produkt auch eine Belagauflösende Wirkung zu. Dies betrifft insbesondere Biofilme und fibrinöse Beläge.

Die folgende Tabelle zeigt beispielhafte Eigenschaften einer bevorzugten flächigen Lage. Dabei sind die Wertebereiche einschließlich der die Wertebereiche eingrenzenden Zahlenwert zu verstehen.

| **Parameter** | **Bevorzugt** | **Sehr bevorzugt** | **Besonders bevorzugt** |
|---|---|---|---|
| Flächengewicht (g/m² ) | 100-900 | 450 - 750 | 550 - 600 |
| Dicke (mm) | 1 - 10 | 2-8 | 3,5 - 4,5 |
| Aufnahmekapazität 0.9 % Kochsalzlösung (g/g) | 10 - 100 | 20-70 | 30 - 40 |
| Aufnahmekapazität demineralisiertes Wasser (g/g) | 40 - 400 | 60 - 200 | 80 - 100 |

Dabei ist bevorzugt vorgesehen, dass die flächige Lage im Randbereich dünner ausfällt, sich also beispielsweise im Querschnitt an den Rändern konisch verjüngt. Damit ist gewährleistet, dass an den Wundrändern ein geringerer Auftrag vorliegt.

Ferner ist bevorzugt vorgesehen, dass die flächige Lage mindestens auf einer Seite von einem dünnen Nonwoven be- oder unterlegt ist. Hierbei kann es sich beispielsweise um ein dünnes, wasserdurchlässiges Vlies aus Polypropylen, Polyethylen oder Polyester handelt. Dessen Flächengewicht liegt bevorzugt im Bereich zwischen 5 - 20 g/m². Ein solches Nonwoven verbessert den strukturellen Zusammenhalt der Lage insbesondere nach der Aufnahme von Flüssigkeit.

Der erfindungsgemäße Wundpflegeartikel kann ferner mindestens eine flächige Lage aufweisend Zellulosefasern, Schaumstoff, modifizierte Cellulose und/oder Alginate aufweisen.

Der Begriff "Schaumstoff" bezeichnet einen offen- oder geschlossenporigen Schaumstoff, bevorzugt aus Polyurethan.

Bei modifizierter Cellulose handelt es sich bevorzugt um Derivate der Cellulose, bevorzugt Nanocellulose, sulfonierte und/oder sulfoalkylierte Cellulose und deren Derivate, bevorzugt Celluloseethylsulfonate, carboxyalkylierte Cellulose, bevorzugt Carboxymethylzellulose, Carboxyethylcellulose und/oder Carboxypropylcellulose, komplexere Cellulosederivative, wie Sulphoethylcarboxymethylcellulose, Carboxymethylhydroxyethylcellulose, Hydroxypropyl-methylcellulose, und amidierte Cellulosederivate, wie Carboxymethylcellulose-Amid oder Carboxypropylcellulose-Amid. Carboxymethylcellulose liegt insbesondere in Form von Natriumcarboxymethylcellulose vor und ist unter dem Namen "Hydrofaser" im Handel. In Hygiene- und Wundprodukten werden die Fasern in eine flächige Matrix überführt. Durch die Aufnahme von Flüssigkeit aus dem Wundexsudat werden die Fasern nach und nach in ein Gelkissen umgewandelt, das die Flüssigkeit hält und nicht wieder freigibt. Dabei sind die Fasern so aufgebaut, dass das Wundexsudat nur in vertikaler Richtung aufgenommen wird. Dies bedeutet, dass, solange die Kapazität reicht, das Exsudat nicht über den Wundrand fließt. Auf diese Weise kann eine Wundrandmazeration effektiv verhindert werden. Auch Chitine, Chitosane und deren Derivate sollen im vorliegenden Kontext als Cellulosederivate verstanden werden.

Alginate werden aus den Braunalgen gewonnen und zu einem faserigen Vlies verwoben. Chemisch handelt es sich um Polysaccharide, und zwar Calcium- und/oder Natriumsalze der Alginsäuren. Alginate können bis zum 20-fachen ihres Eigengewichtes an Flüssigkeit aufnehmen, dabei wird das Wundexsudat in die Hohlräume eingelagert. Die im Alginatgitter enthaltenen Ca²⁺ Ionen werden gegen die Na⁺ Ionen aus dem Exsudat ausgetauscht, bis der Sättigungsgrad an Na-Ionen im Alginat erreicht ist. Dabei kommt es zu einem Aufquellen der Wundauflage und zur Umwandlung der Alginatfaser in einen Gelkörper durch Aufquellen der Fasern.

Weiterhin ist bevorzugt vorgesehen, dass der Wundpflegeartikel eine Hülle aufweist, die mindestens partiell aus einem flüssigkeitsdurchlässigen Material besteht. Eine solche Hülle weist eine mannigfaltige Funktion auf. Sie kann u.a. das Verkleben des Wundpflegeartikels mit der Wunde verhindern, einen Rückfluss von Exsudat in die Wunde verhindern, hypoaller- gen wirken und eine Wundrandmazeration verhindern. Die Hülle ist bevorzugt wenigstens teilweise von einer Naht abgeschlossen, beispielsweise einer Klebenaht oder einer Ultraschallnaht, und kann sowohl aus einer Folie oder einem Film (beispielsweise aus PE) als auch aus einem Nonwoven (beispielsweise aus PP) oder Fleece bestehen.

Insbesondere kann vorgesehen sein, dass die Hülle Poren aufweist, die im Mittel kleiner sind als die Superabsorbierenden Partikel. Auf diese Weise kann ein Herausrieseln von Partikel aus der Hülle vermieden werden. Letzteres kann insbesondere den Sterilisationsprozess behindern, nämlich dann, wenn Partikel in den Bereich der Siegelbarriere gelangen und dort ggf. Undichtigkeiten verursachen.

Die Hülle kann auch mit einem Schwermetall in elementarer oder Ionenform beschichtet oder versetzt sein, beispielsweise Silber, Zink oder Kupfer. Die Hülle kann auch mit einem Material beschichtet sein, das durch hydrophobe Interaktionen Bakterien bindet, wie beispielsweise Dialkylcarbamoylchloride (DACC).

Die Porengröße beeinträchtigt jedoch auch die Durchflussgeschwindigkeit des aufzunehmenden Exsudats. Dies trifft insbesondere für mehr oder minder hydrophobe Polymermaterialien zu. Durch eine geeignete Avivage-Behandlung kann allgemein die Benetzbarkeit der Hülle verbessert und dadurch auch die Durchflussgeschwindigkeit auch bei kleinen Poren sichergestellt werden.

Die Poren oder Maschen der Hülle sind bevorzugt 0,05 mm bis 1,0 mm, vorzugsweise 0,20 mm bis 0,50 mm gross. Weiterhin kann bevorzugt vorgesehen sein, dass die Poren oder Maschen durch die Fäden- oder Faserabschnitte begrenzt sind, welche im Schnitt durch die Hülle etwa bogenförmig sind und mit ihren Bogen-Scheiteln nach außen zeigen.

Bevorzugt ist dabei vorgesehen, dass die flächige Lage
(i) in Draufsicht auf ihre Flachseite eine Fläche (F1) aufweist, welche im nicht benetzten Zustand um 3% bis 75% kleiner als die Fläche (F2) des von der Hülle bereitgestellten Innenraums ist, und/oder
(ii) die Hülle mindestens abschnittsweise ein elastisches Material aufweist.

Im erstgenannten Fall ist von einem sogenannten Expansionsraum, die Rede, welchen die Hülle der flächigen Lage zur Verfügung stellt. In beiden Fällen wird so Sorge getragen, dass die Hülle der durch die Aufnahme von Flüssigkeit bedingten Volumenzunahme der flächigen Lage keinen Widerstand entgegensetzt, und letztere so ihre volle Absorptionskapazität ausschöpfen kann. Das elastische Material kann beispielsweise Lycra, Elasthan, Polypropylen, Kautschuk, Latex, Nylon oder ähnliches aufweisen.

Ferner ist bevorzugt vorgesehen, dass die Hülle mindestens partiell aus einem dreidimensionalen Wunddistanzgitter besteht bzw. von diesem be- oder unterlegt ist. Bei besagtem Wunddistanzgitter handelt es sich bevorzugt um ein aus einer Polyethylenfolie durch Blasverfahren erzeugtes Wunddistanzgitter, wie beispielsweise in der EP2004116A1 beschrieben. Ebenso kann es sich beispielsweise um ein Silikongitter handeln oder auch um ein Nylongitter oder eine Gaze.

Ein solches Gitter hat mannigfaltige Funktionen. Es kann, je nach Ausgestaltung der Poren, eine Ventilfunktion ausüben und so den Rückfluss von Exsudat verhindern (bevorzugt bei trichter- oder kragenförmiger Ausgestaltung der Poren). Es kann verhindern, dass der Wundpflegeartikel mit der Wunde verklebt (bevorzugt bei Verwendung eines Silikonmaterials). Es kann bei geeigneter Anordnung abrasive Eigenschaften ausbilden und so die Biofilme auf der Wunde abtragen bzw. deren Entstehung verhindern (bevorzugt bei trichter- oder kragenförmiger Ausgestaltung der Poren). Es kann ferner Blutstillende Eigenschaften aufweisen und ggf. auch imstande sein, Bakterien durch statische Wechselwirkungen zu immobilisieren bzw. zu binden (bevorzugt bei Verwendung eines Polyethylenmaterials oder eines Materials mit positiver Nettoladung). Ferner kann die Oberfläche funktionalisiert sein, beispielsweise mit einer Silber-oder Silikonbeschichtung. Bevorzugt ist ferner vorgesehen, dass die Hülle mindestens partiell aus einem imprägnierten bzw. wasserundurchlässigen Material besteht bzw. von diesem be- oder unterlegt ist. Hierbei kann es sich um einen farblich ggf. auffällig gestalteten Wäscheschutz ("Backsheet") handeln.

Bevorzugt ist ferner vorgesehen, dass der Wundpflegeartikel einen Anteil an mindestens einem elementaren oder in Ionenform vorliegenden Schwermetall aufweist. Schwermetalle wirken in feinstverteilter Form bakterizid, was aufgrund der großen reaktiven Oberfläche auf die hinreichende Entstehung von löslichen Schwermetallionen zurückzuführen ist.

Durch die Dotierung mit mindestens einem elementaren oder in Ionenform vorliegenden Schwermetall kann dem Primärverband eine antibakterielle Wirkung verliehen werden, was Komplikationen bei infektionsgefährdeten Wunden (Dekubitus, Ulcus Cruris, Verbrennungswunden, etc.) vermindert und gleichzeitig die Verweildauer der Wundauflage erhöhen kann.

Bevorzugt ist dabei vorgesehen, dass das mindestens eine elementare oder in Ionenform vorliegenden Schwermetall ausgewählt ist aus der Gruppe enthaltend Kupfer, Zink und/oder Silber. Die oben genannten bakteriziden Eigenschaften gelten in besonderer Form für diese drei Metalle.

Ferner ist bevorzugt vorgesehen, dass die flächige Lage oder die Hülle mindestens auf einer Seite durch eine Abdeckfolie belegt oder ersetzt ist. Bevorzugt weist besagte Abdeckfolie mindestens eine der nachfolgenden Eigenschaften auf:
- Adhäsive Beschichtung
- flüssigkeitsdicht
- wasserdampfdurchlässig und/oder
- elastisch.

Dabei ist besonders bevorzugt vorgesehen, dass die Abdeckfolie über eine Peripherie des Wundpflegeartikels hinausragt und an der Umgebungshaut der Wunde anbringbar ist. Auf diese Weise ist ein sogenanntes Border- oder Island-Dressing verwirklicht.

Alternativ ist vorgesehen, dass die Hülle selbst mindestens auf einer Seite eine adhäsive Beschichtung aufweist. In den genannten Fällen weist die adhäsive Beschichtung bevorzugt einen Acrylatkleber, einen Silikonkleber, einen Stärkekleber, einen Hydrokolloidkleber und/oder einen anderen geeigneten physiologisch unbedenklichen Kleber auf.

Besagte Abdeckfolie oder das zuvor erwähnte Backsheet kann mit der darunterliegenden Lage verklebt sein. Dabei kommt bevorzugt ein elastischer Kleber zum Einsatz, um die Volumenzunahme des Produkts bei Flüssigkeitsaufnahme zu ermöglichen. Alternativ kann aber auch vorgesehen sein, dass die Abdeckfolie oder das Backsheet nicht mit der darunterliegenden Lage verklebt ist.

Ferner ist erfindungsgemäß bevorzugt vorgesehen, dass der mindestens ein Befestigungselement zur Fixierung desselben an einem Körperteil aufweist. Hierbei kann es sich beispielsweise um einen oder mehrere Klettverschlüsse, einen oder mehrere Klebeverschlüsse, eine oder mehrere Bandagenklammern oder dergleichen handeln. In bevorzugten Ausgestaltungen können diese Befestigungselemente auch als Kompressionselemente ausgebildet sein, beispielsweise als Kompressionsbänder oder - Riemen, um auf diese Weise einen Kompressionsverband bereitzustellen.

Der Wundpflegeartikel kann ferner mindestens einen Bestandteil ausgewählt aus der Gruppe enthaltend
- Hyaluronsäure (bevorzugt als Ummantelung zu superabsorbierenden Polymeren)
- Octenidin
- Dimeticon
- Aktivkohle
aufweisen.

Weiterhin ist bevorzugt vorgesehen, dass der Wundpflegeartikel ein oder mehrere Faltlinien, Umbruchlinien oder Falze aufweist. Dies ist insbesondere dann hilfreich, wenn der Wundpflegeartikel eine gewisse Mindestgröße aufweist. Mithilfe besagter Faltlinien, Umbruchlinien oder Falze kann der Wundpflegeartikel in einem platzsparenden zusammengefalteten Zustand verpackt, sterilisiert, gelagert und transportiert werden. Erst beim Entnehmen aus der Verpackung wird der Wundpflegeartikel dann zu seiner vollen Größe aufgefaltet.

Ferner ist ein erfindungsgemäßer Wundpflegeartikel zur Anwendung in einem Verfahren zur Wundbehandlung durch
- Anlage an einem Ober- oder Unterschenkel
- Anlage im Schulter-, Brust- oder Nackenbereich
- Anlage im Achselbereich
- Anlage im Unter- oder Oberbrustbereich
- Anlage im Rippenbogenbereich
- Anlage im Unter- oder Oberarmbereich
- Anlage im Unteraugenbereich
- Anlage im Sacralbereich
- Anlage am Rücken
- Anlage in der Kniekehle
- Anlage im Unterbauchbereich
- Anlage im Hinter- oder Oberohrbereich
- Anlage im Interdigitalbereich und/oder
- Anlage im Fussbereich
eines Patienten vorgesehen.

Dabei werden je nach Anlageort von der erfindungsgemäßen Ausnehmung bestimmte Körperbereiche ausgespart, was wiederum zu einer hervorragenden Anpassung an die anatomischen Gegebenheiten führt.

| **Anlageort** | **Von der Ausnehmung ausgesparter Bereich** | **Indikation (beispielhaft)** | **Bemassung der Grundfläche (beispielhaft)** |
|---|---|---|---|
| Unterschenkel | Spann (Fußrücken) | Behandlung des sogenannten Gamaschenulcus | 235 x 435 mm |
| Oberschenkel | Kniebeuge | Verbrennungen, post-OP-Versorgung, plastische Chirurgie | 280 x 500 mm |
| Schulter-, Brust- oder Nackenbereich | Hals | Verbrennungen, post-OP-Versorgung, plastische Chirurgie | 280 x 500 mm |
| Achselbereich | Achsel | Verbrennungen, post-OP-Versorgung, plastische Chirurgie | 180 x 360 mm |
| Unterbrustbereich | Brust | Verbrennungen, post-OP-Versorgung, plastische Chirurgie | 180 x 360 mm |
| Oberbrustbereich | Brustbein und Plexus | Verbrennungen, post-OP-Versorgung, plastische Chirurgie | 180 x 360 mm |
| Rippenbogenbereich | Rippenbogenbereich | Verbrennungen, post-OP-Versorgung, plastische Chirurgie | 235 x 435 mm |
| Unterarmbereich | Ellenbeuge | Verbrennungen, post-OP-Versorgung, plastische Chirurgie | 200 x 400 mm |
| Oberarm bereich | Achsel | Verbrennungen, post-OP-Versorgung, plastische Chirurgie | 235 x 435 mm |
| Unteraugenbereich | Augenhöhlen | Verbrennungen, post-OP-Versorgung, plastische Chirurgie | 120 x 65 mm |
| Sacralbereich | Gesäßspalte | Decubitus | 200 x 400 mm |
| Rücken | Hals | Verbrennungen, post-OP-Versorgung, plastische Chirurgie | 235 x 435 mm |
| Unterbauchbereich | Schambereich | Verbrennungen, post-OP-Versorgung, plastische Chirurgie, nach Luposuction | 180 x 360 mm |
| Hinter- oder Oberohrbereich | Ohr | Verbrennungen, post-OP-Versorgung, plastische Chirurgie, nach Luposuction | 120 x 65 mm |
| Interdigitalbereich | Zehen bzw. Finger | Verbrennungen, post-OP-Versorgung, plastische Chirurgie, nach Luposuction | |
| Fußbereich | Spann und Fußgelenk | Verbrennungen, post-OP-Versorgung, plastische Chirurgie, nach Luposuction | |

Ferner ist ein erfindungsgemäßer Wundpflegeartikel zur Verwendung in einem Verfahren zur Wundbehandlung durch Verwendung in einem Unterdruck- Wundversorgungssystem vorgesehen.

Weitere Vorteile und vorteilhafte Ausgestaltungen der erfindungsgemäßen Gegenstände werden durch die Zeichnungen veranschaulicht und in der nachfolgenden Beschreibung erläutert. Dabei ist zu beachten, dass die Zeichnungen nur beschreibenden Charakter haben und nicht dazu gedacht ist, die Erfindung in irgendeiner Form einzuschränken.

Es zeigt:

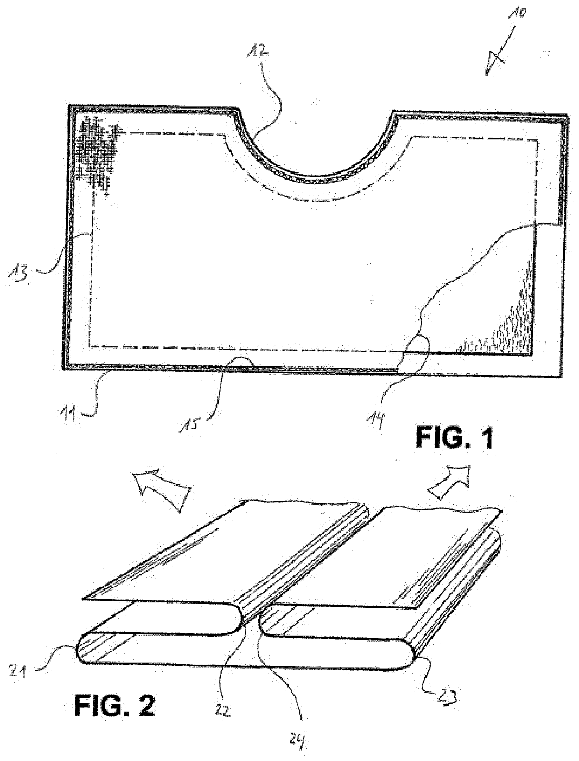

Fig. 1 einen flächigen Wundpflegeartikel 10 aufweisend eine im wesentlichen polygonale Grundfläche 11 sowie eine an einer Seite angeordnete halbkreisförmige Ausnehmung 12. Die Grundfläche ist rechteckig, und die Ausnehmung ist an einer Längsseite angeordnet. Der Wundpflegeartikel weist durch diese recht einfache Modifikation eine sehr viel höhere Variabilität auf und erhält insbesondere eine gute Anpassbarkeit an die anatomischen Bedingungen verschiedener Körperregionen von Mensch und Tier.

Der Wundpflegeartikel weist eine flächige Lage 13 aufweisend ein absorbierendes Material auf, die aus einer flächigen Lage aufweisend ein superabsorbierendes Polymer besteht.

Der Wundpflegeartikel weist ferner eine Hülle 14 auf, die mindestens partiell aus einem flüssigkeitsdurchlässigen Material besteht, und von einer Naht 15 abgeschlossen ist oder einer Ultraschallnaht. Die flächige Lage weist in Draufsicht auf ihre Flachseite eine Fläche (F1) auf, welche im nicht benetzten Zustand um 3% bis 75% kleiner als die Fläche (F2) des von der Hülle bzw. der Hüllennaht bereitgestellten Innenraums ist.

Hierdurch wird ein Expansionsraum gebildet, welcher dafür Sorge trägt, dass die Hülle der durch die Aufnahme von Flüssigkeit bedingten Volumenzunahme der flächigen Lage keinen Widerstand entgegensetzt, und letztere so ihre volle Absorptionskapazität ausschöpfen kann.

Fig. 2 zeigt den besagten Wundpflegeartikel in einem zusammengelegten Zustand. Hierzu weist er wie im vorliegenden Beispiel gezeigt, vier Umbruchlinien 21, 22, 23 und 24 auf, mittels derer in einem platzsparenden zusammengefalteten Zustand verpackt, sterilisiert, gelagert und transportiert werden. Erst beim Entnehmen aus der Verpackung wird der Wundpflegeartikel dann zu seiner vollen Größe aufgefaltet, wie durch die beiden Pfeile angedeutet.

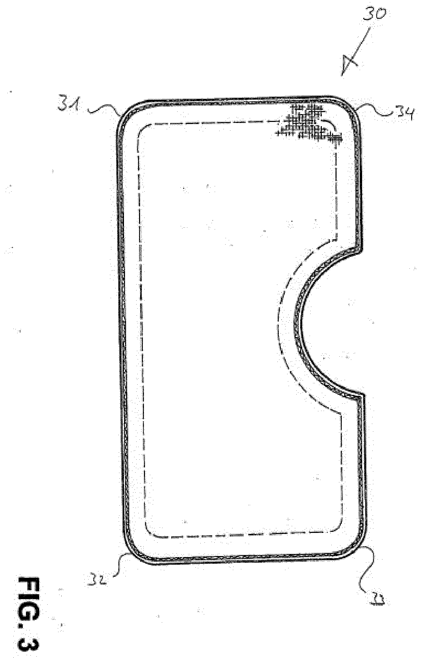

Fig. 3 zeigt einen flächigen Wundpflegeartikel 30, der im Unterscheid zu dem in Fig. 1 gezeigten Wundpflegeartikel abgerundete Ecken 31, 32, 33 und 34 aufweist.

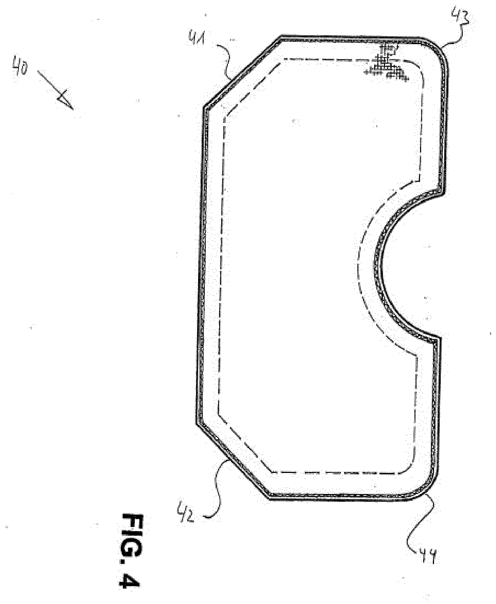

Fig. 4 zeigt einen flächigen Wundpflegeartikel 40, der zwei abgewinkelte Ecken 41, 42 und zwei abgerundete Ecken 43, 44 aufweist.

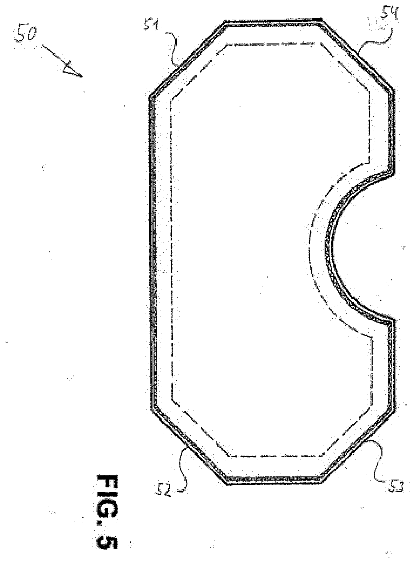

Fig. 5 zeigt einen flächigen Wundpflegeartikel 50, der abgewinkelte Ecken 51, 52, 53 und 54 aufweist.

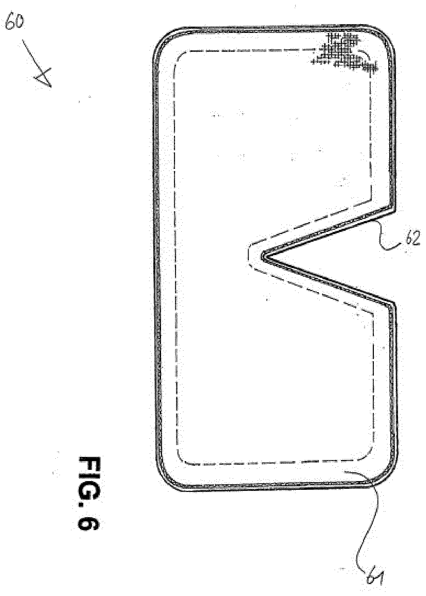

Fig. 6 zeigt einen flächigen Wundpflegeartikel 60, aufweisend eine im wesentlichen polygonale Grundfläche 61 mit abgerundeten Ecken sowie eine an einer Seite angeordnete winkelförmige Ausnehmung 62. Die Grundfläche ist rechteckig, und die Ausnehmung ist an einer Längsseite angeordnet.

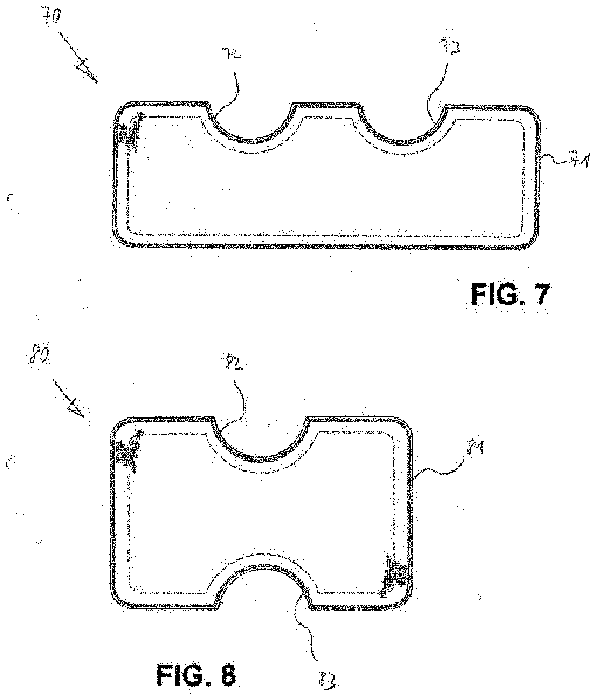

Fig. 7 zeigt einen flächigen Wundpflegeartikel 70, aufweisend eine im wesentlichen polygonale Grundfläche 71 mit abgerundeten Ecken sowie zwei eine an einer Seite angeordnete halbkreisförmige Ausnehmungen 72 und 73. Die Grundfläche ist rechteckig, und die Ausnehmungen sind an einer Längsseite angeordnet.

Fig. 8 zeigt einen flächigen Wundpflegeartikel 80, aufweisend eine im wesentlichen polygonale Grundfläche 81 mit abgerundeten Ecken sowie zwei an den jeweils gegenüberliegenden Längsseiten angeordnete halbkreisförmige Ausnehmungen 82 und 83.

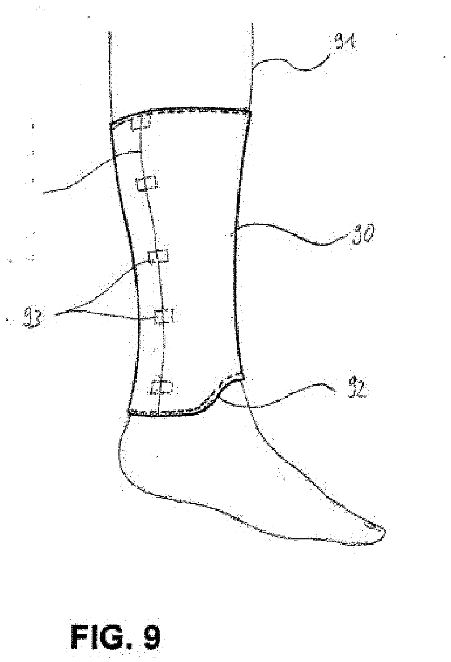

Fig. 9 zeigt einen flächigen Wundpflegeartikel 90 in Anlage am Unterschenkel 91 eines Patienten. Ein solcher Wundpflegeartikel eignet sich beispielsweise zur Behandlung eines sogenannten Gamaschenulcus. Der Wundpflegeartikel weist eine halbkreisförmige Ausnehmung auf, die den Spann (Fußrücken) des Patienten ausspart und so den anatomischen Bedingungen der betreffenden Körperregion Rechnung trägt. Der Wundpflegeartikel weist ferner Klettbänder 93 zur Fixierung desselben am Unterschenkel des Patienten auf, die aber auch als Kompressionsbänder verwendet werden können, um auf diese Weise einen Kompressionsverband herzustellen.

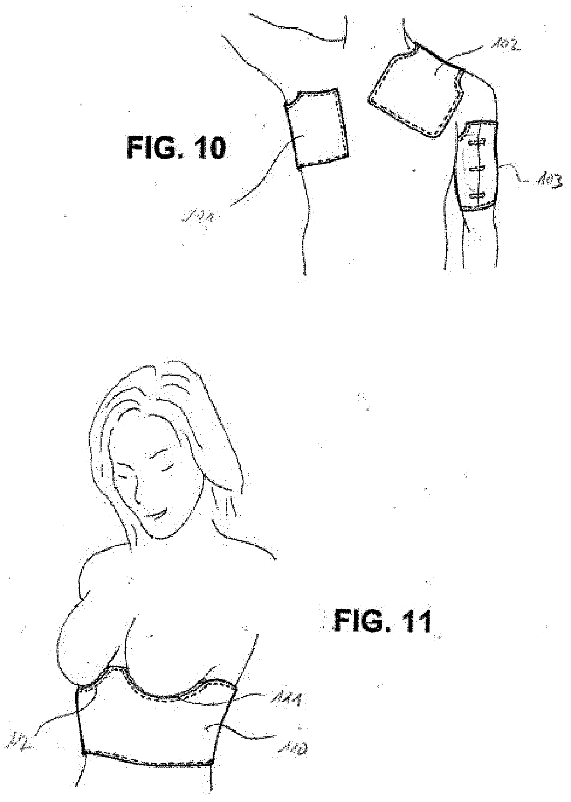

Fig. 10 zeigt einen flächigen Wundpflegeartikel 101, 102, 103 in Anlage am Achselbereich, Schulterbereich oder Oberarmbereich eines Patienten. Der Wundpflegeartikel weist eine halbkreisförmige Ausnehmung auf, die die Achsel bzw. den Hals des Patienten ausspart und so den anatomischen Bedingungen der betreffenden Körperregion Rechnung trägt.

Fig. 11 zeigt einen flächigen Wundpflegeartikel 110 in Anlage am Brustbereich einer Patientin. Der Wundpflegeartikel weist zwei halbkreisförmige Ausnehmungen 111, 112 auf, die die Brust der Patientin aussparen und so den anatomischen Bedingungen der betreffenden Körperregion Rechnung tragen.

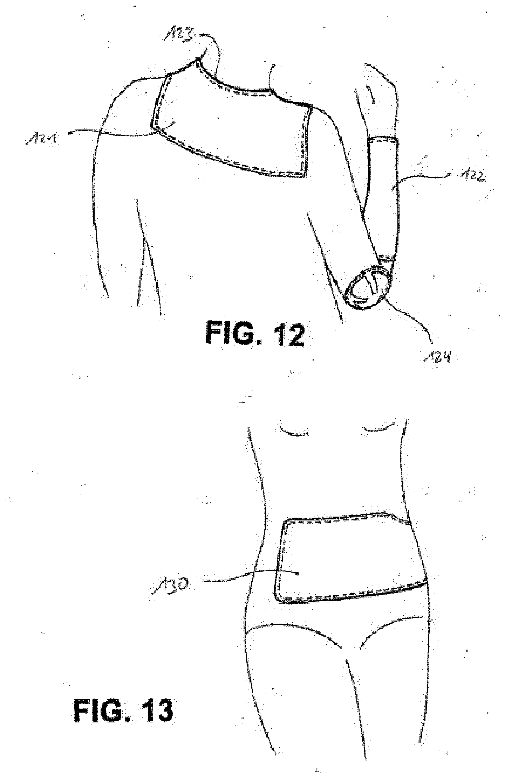

Fig. 12 zeigt einen flächigen Wundpflegeartikel 121, 122 in Anlage am Nackenbereich oder Unterarm eines Patienten Der Wundpflegeartikel weist eine halbkreisförmige Ausnehmung auf, die den Hals 123 bzw. die Ellenbeuge des Patienten ausspart und so den anatomischen Bedingungen der betreffenden Körperregion Rechnung trägt. Fig. 12 zeigt ferner einen weiteren Wundpflegeartikel 124 der Anmelderin in Anlage am Ellenbogen des Patienten.

Fig. 13 zeigt einen flächigen Wundpflegeartikel 130 in Anlage am Rippenbogenbereich eines Patienten. Der Wundpflegeartikel weist eine halbkreisförmige Ausnehmung auf, die den oberen Rippenboden des Patienten ausspart und so den anatomischen Bedingungen der betreffenden Körperregion Rechnung trägt.

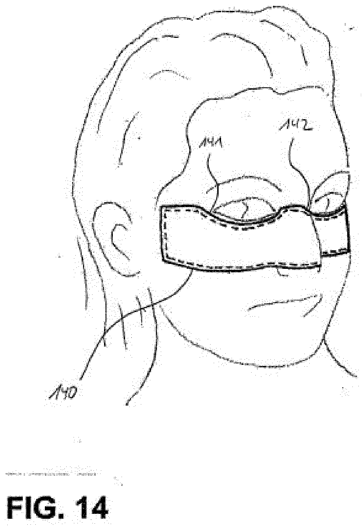

Fig. 14 zeigt einen flächigen Wundpflegeartikel 140 in Anlage am Augenbereich eines Patienten. Der Wundpflegeartikel weist zwei halbkreisförmige Ausnehmungen 141, 142 auf, die die Augenhöhlen des Patienten aussparen und so den anatomischen Bedingungen der betreffenden Körperregion Rechnung tragen.

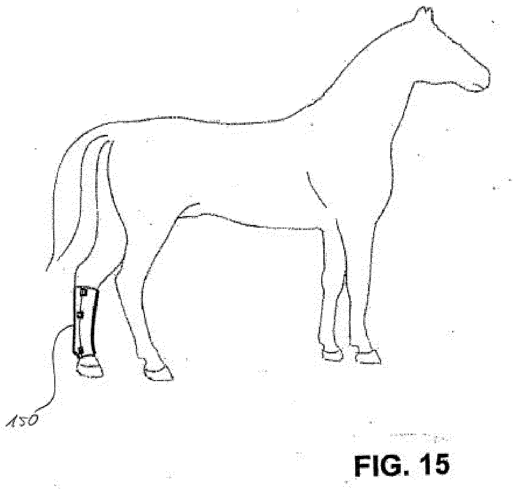

Fig. 15 zeigt einen flächigen Wundpflegeartikel 150 in Anlage am Bein eines Pferdes. Der Wundpflegeartikel weist eine halbkreisförmige Ausnehmung auf, die den Huf des Pferdes ausspart und so den anatomischen Bedingungen der betreffenden Körperregion Rechnung trägt. Der Wundpflegeartikel weist ferner Klettbänder zur Fixierung desselben am Bein des Pferdes auf.

Fig. 16 zeigt einen flächigen Wundpflegeartikel 161 in Anlage am Achselbereich eines Patienten. Der Wundpflegeartikel weist eine halbkreisförmige Ausnehmung auf, die die Achsel bzw. den Hals des Patienten ausspart und so den anatomischen Bedingungen der betreffenden Körperregion Rechnung trägt.

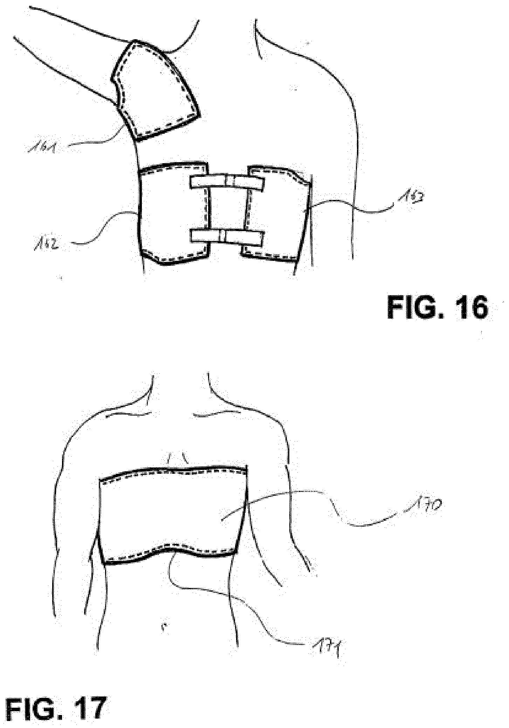

Fig. 16 zeigt ferner zwei flächige Wundpflegeartikel 162, 163, in Anlage an beiden Rippenbogenbereichen eines Patienten. Die Wundpflegeartikel weisen eine halbkreisförmige Ausnehmung auf, die den oberen Rippenboden bzw. die Achsel des Patienten ausspart und so den anatomischen Bedingungen der betreffenden Körperregion Rechnung trägt.

Fig. 17 zeigt einen flächigen Wundpflegeartikel 170 in Anlage am Brustbereich eines Patienten. Der Wundpflegeartikel weist eine halbkreisförmige Ausnehmung 171 auf, die den Plexusbereich und das Brustbein des Patienten ausspart und so den anatomischen Bedingungen der betreffenden Körperregion Rechnung trägt.

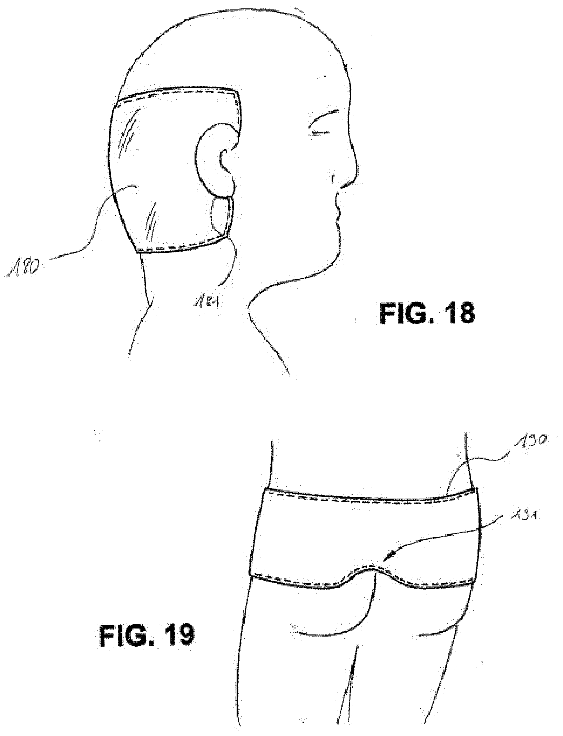

Fig. 18 zeigt einen flächigen Wundpflegeartikel 180 in Anlage am Hinterkopf eines Patienten. Der Wundpflegeartikel weist eine halbkreisförmige Ausnehmung 181 auf, die das Ohr des Patienten ausspart und so den anatomischen Bedingungen der betreffenden Körperregion Rechnung trägt.

Fig. 19 zeigt einen flächigen Wundpflegeartikel 190 in Anlage am Sakralbereich eines Patienten. Der Wundpflegeartikel weist eine halbkreisförmige Ausnehmung 191 auf, die die Gesäßspalte des Patienten ausspart und so den anatomischen Bedingungen der betreffenden Körperregion Rechnung trägt.

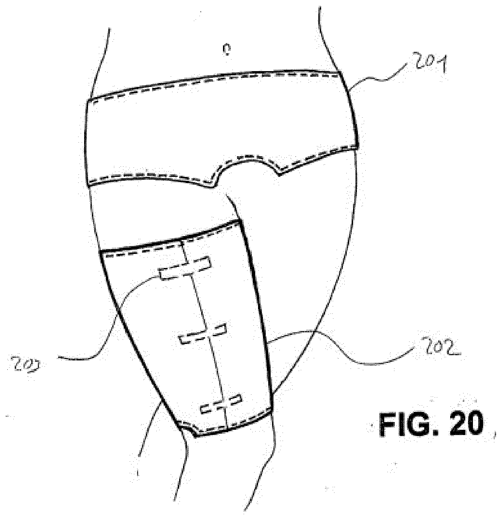

Fig. 20 zeigt einen flächigen Wundpflegeartikel 201 in Anlage im Unterbauchbereich eines Patienten. Der Wundpflegeartikel weist eine halbkreisförmige Ausnehmung auf, die den Schambereich des Patienten ausspart und so den anatomischen Bedingungen der betreffenden Körperregion Rechnung trägt. Die Grundfläche des Wundpflegeartikels ist dabei aus einem Trapez abgeleitet.

Fig. 20 zeigt ferner einen flächigen Wundpflegeartikel 202 in Anlage am Oberschenkel eines Patienten. Der Wundpflegeartikel weist eine halbkreisförmige Ausnehmung auf, die die Kniebeuge des Patienten ausspart und so den anatomischen Bedingungen der betreffenden Körperregion Rechnung trägt. Ferner wiest der Wundpflegeartikel Klettverschlüsse 203 auf, die zur Sicherung des Wundpflegeartikels dienen, aber auch als Kompressionsbänder verwendet werden können, um auf diese Weise einen Kompressionsverband herzustellen.

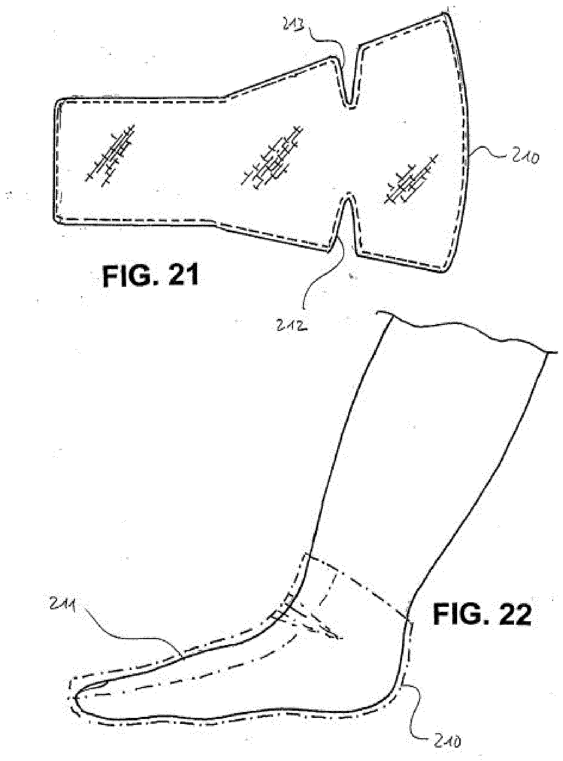

Figuren 21 und 22 zeigen einen flächigen Wundpflegeartikel 210 in Anlage am Fuß 211 eines Patienten. Der Wundpflegeartikel weist zwei V-förmige Ausnehmungen 212, 213 auf, die den Fußspann des Patienten aussparen und so den anatomischen Bedingungen der betreffenden Körperregion Rechnung tragen.

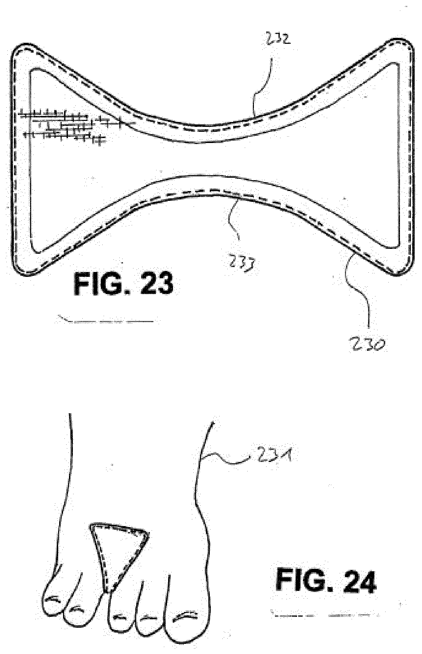

Figuren 23 und 24 zeigen einen flächigen Wundpflegeartikel 230 in Anlage im Zehenzwischenraum am Fuß 231 eines Patienten. Der Wundpflegeartikel weist zwei halbkreisförmige Ausnehmungen 232, 233 auf, die die Zehen des Patienten aussparen und so den anatomischen Bedingungen der betreffenden Körperregion Rechnung tragen. Auf diese Weise ist ein Interdigital verband verwirklicht, der Beispielsweise auch an der Hand eines Patienten angelegt werden kann.

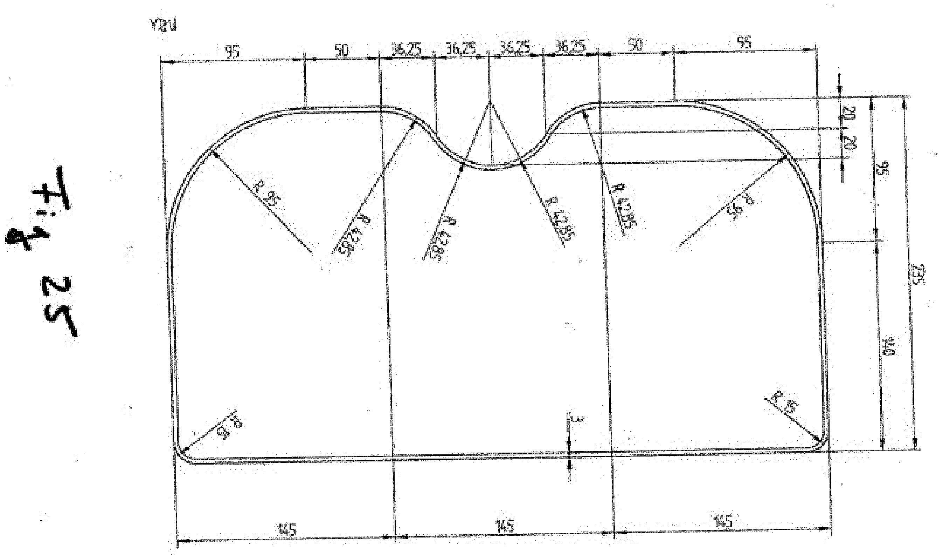

Fig. 25 zeigt einen flächigen Wundpflegeartikel gemäß der Erfindung mit beispielhaften Bemaßungen.

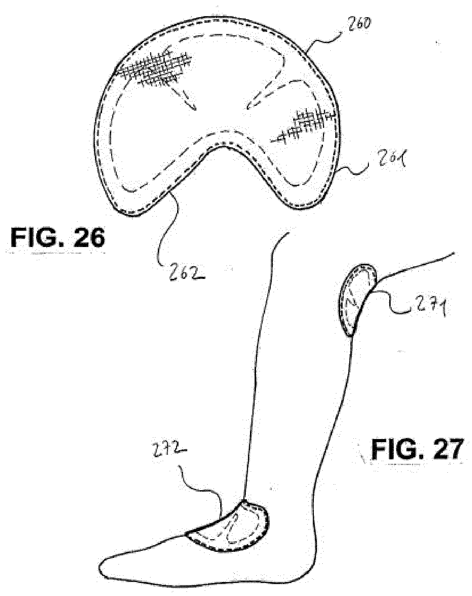

Fig. 26 zeigt einen flächigen Wundpflegeartikel 260 aufweisend eine im wesentlichen ellipsoide Grundfläche 261 sowie eine an einer Seite angeordnete winkelförmige Ausnehmung 262.

Fig. 27 zeigt besagten Wundpflegeartikel 260 in Anlage in der Kniekehle 271 bzw. dem Spann 272 eines Patienten.

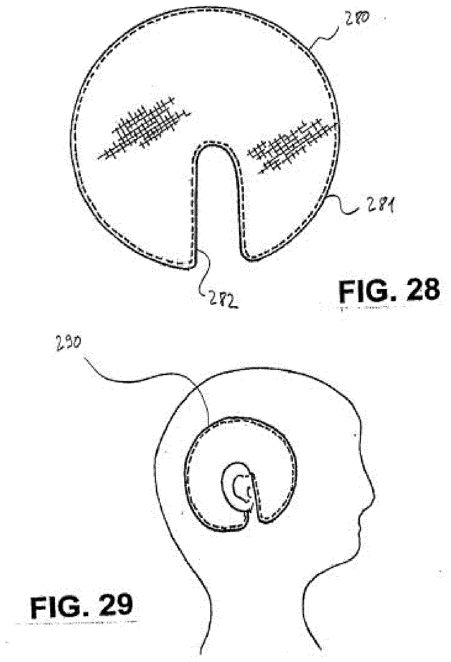

Fig. 28 zeigt einen flächigen Wundpflegeartikel 280 aufweisend eine im wesentlichen kreisförmige Grundfläche 281 sowie eine an einer Seite angeordnete Ausnehmung 282.

Fig. 29 zeigt besagten Wundpflegeartikel 280 in Anlage am Ohr 290 eines Patienten.

## Patentansprüche

1. Flächiger Wundpflegeartikel aufweisend
• eine Grundfläche die ein nicht-gleichseitiges Rechteck ist oder aus einem solchen abgeleitet ist, mindestens eine abgerundete Ecke aufweist, und eine an einer Längsseite angeordnete Ausnehmung, die kreisabschnittsförmig oder ellipsenabschnittsförmig ist;
• eine flächige Lage aufweisend ein superabsorbierendes Polymer,
• eine die flächige Lage umgebende Hülle, die mindestens partiell aus einem flüssigkeitsdurchlässigen Material besteht,
• wobei die flächige Lage in Draufsicht auf ihre Flachseite eine Fläche (F1) aufweist, welche im nicht benetzten Zustand um 3% bis 75% kleiner als die Fläche (F2) des von der Hülle bzw. der Hüllennaht bereitgestellten Innenraums ist.

2. Wundpflegeartikel gemäß Anspruch 1, ferner aufweisend mindestens eine flächige Lage aufweisend Zellulosefasern, Schaumstoff, modifizierte Cellulose und/oder Alginate.

3. Wundpflegeartikel gemäß Anspruch 1, **dadurch gekennzeichnet, dass** die Hülle mindestens abschnittsweise ein elastisches Material aufweist.

4. Wundpflegeartikel gemäß Anspruch 1 - 3, wobei die Hülle mindestens partiell aus einem dreidimensionalen Wunddistanzgitter besteht bzw. von diesem be- oder unterlegt ist.

5. Wundpflegeartikel gemäß Anspruch 1 - 4, wobei die Hülle mindestens partiell aus einem imprägnierten bzw. wasserundurchlässigen Material besteht bzw. von diesem be- oder unterlegt ist.

6. Wundpflegeartikel gemäß einem der vorherigen Ansprüche, aufweisend einen Anteil an mindestens einem elementaren oder in Ionenform vorliegenden Schwermetall.

7. Wundpflegeartikel gemäß Anspruch 6 wobei das mindestens eine elementare oder in Ionenform vorliegenden Schwermetall ausgewählt ist aus der Gruppe enthaltend Kupfer, Zink und/oder Silber.

8. Wundpflegeartikel nach einem der vorherigen Ansprüche, **dadurch gekennzeichnet, dass** die flächige Lage oder die Hülle mindestens auf einer Seite durch eine Abdeckfolie belegt oder ersetzt ist.

9. Wundpflegeartikel gemäß Anspruch 8, **dadurch gekennzeichnet, dass** die Abdeckfolie über eine Peripherie des Wundpflegeartikels hinausragt und an der Umgebungshaut der Wunde anbringbar ist.

10. Wundpflegeartikel nach einem der vorhergehenden Ansprüche, **dadurch gekennzeichnet, dass** die flächige Lage oder die Hülle mindestens auf einer Seite eine adhäsive Beschichtung aufweist.

11. Wundpflegeartikel nach einem der vorhergehenden Ansprüche, ferner aufweisend mindestens ein Befestigungselement zur Fixierung desselben an einem Körperteil.

12. Wundpflegeartikel nach einem der vorhergehenden Ansprüche, ferner aufweisend ein oder mehrere Faltlinien, Umbruchlinien oder Falze.

13. Wundpflegeartikel gemäß einem der vorhergehenden Ansprüche zur Anwendung in einem Verfahren zur Wundbehandlung durch
• Anlage an einem Ober- oder Unterschenkel
• Anlage im Schulter-, Brust- oder Nackenbereich
• Anlage im Achselbereich
• Anlage im Unter- oder Oberbrustbereich
• Anlage im Rippenbogenbereich
• Anlage im Unter- oder Oberarmbereich
• Anlage im Unteraugenbereich
• Anlage im Sacralbereich
• Anlage am Rücken
• Anlage in der Kniekehle
• Anlage im Unterbauchbereich
• Anlage im Hinter- oder Oberohrbereich
• Anlage im Interdigitalbereich und/oder
• Anlage im Fussbereich
eines Patienten.

14. Wundpflegeartikel gemäß einem der vorhergehenden Ansprüche zur Verwendung in einem Verfahren zur Wundbehandlung durch Verwendung in einem Unterdruck-Wundversorgungssystem.

## Claims

1. Flat wound care article comprising
• a main surface which is a non-equilateral rectangle or is derived therefrom, has at least one rounded corner, and a recess arranged on a longitudinal side, which recess is circular-segment-shaped or elliptical-segment-shaped;
• a flat layer comprising a super-absorbent polymer,
• a shell surrounding the flat layer, which shell consists at least partially of a liquid-permeable material,
• wherein the flat layer has, in a plan view of its flat side, a surface area (F1) which, in the non-wetted state, is 3% to 75% smaller than the surface area (F2) of the interior provided by the shell or the shell seam.

2. Wound care article according to claim 1, further comprising at least one flat layer comprising cellulose fibers, foam, modified cellulose and/or alginates.

3. Wound care article according to claim 1, **characterized in that** the shell comprises an elastic material at least in sections.

4. Wound care article according to claims 1-3, wherein the shell at least partially consists of or is coated or underlaid by a three-dimensional wound spacer lattice.

5. Wound care article according to claims 1-4, wherein the shell at least partially consists of or is coated or underlaid by an impregnated or water-impermeable material.

6. Wound care article according to any of the preceding claims, comprising a proportion of at least one heavy metal present in elemental or ionic form.

7. Wound care article according to claim 6, wherein the at least one heavy metal that is present in elemental or ionic form is selected from the group comprising copper, zinc and/or silver.

8. Wound care article according to any of the preceding claims, **characterized in that** the flat layer or the shell is coated or replaced at least on one side by a cover film.

9. Wound care article according to claim 8, **characterized in that** the cover film protrudes beyond a periphery of the wound care article and can be attached to the surrounding skin of the wound.

10. Wound care article according to any of the preceding claims, **characterized in that** the flat layer or the shell has an adhesive coating at least on one side.

11. Wound care article according to any of the preceding claims, further comprising at least one fastening element for fixing said wound care article to a body part.

12. Wound care article according to any of the preceding claims, further comprising one or more fold lines, break lines or seams.

13. Wound care article according to any of the preceding claims for use in a method for treating wounds by
• application on an upper or lower leg
• application in the shoulder, chest or neck area
• application in the armpit area
• application in the lower or upper chest area
• application in the costal arch area
• application in the lower or upper arm area
• application in the infraorbital area
• application in the sacral area
• application on the back
• application in the knee pit
• application in the lower abdomen area
• application in the back or upper ear area
• application in the interdigital area and/or
• application in the foot area
of a patient.

14. Wound care article according to any of the preceding claims for use in a method for treating wounds by use in a vacuum wound care system.

## Revendications

1. Article plan pour le soin des plaies présentant
• une surface de base qui est un rectangle non équilatéral ou est dérivée d'un tel, présente au moins un coin arrondi et un évidement disposé sur un côté longitudinal, qui est en forme de segment de cercle ou en formé de segment d'ellipse ;
• une couche plane présentant un polymère superabsorbant,
• une enveloppe entourant la couche plane, qui se compose au moins partiellement d'un matériau perméable aux liquides,
• la couche plane présentant, en vue de dessus sur sa surface plane, une surface (F1) laquelle est, à l'état non mouillé, de 3 % à 75 % plus petite que la surface (F2) de l'espace intérieur fourni par l'enveloppe ou le joint de l'enveloppe.

2. Article pour le soin des plaies selon la revendication 1, présentant en outre au moins une couche plane présentant des fibres cellulosiques, de la mousse, de la cellulose modifiée et/ou des alginates.

3. Article pour le soin des plaies selon la revendication 1, **caractérisé en ce que** l'enveloppe présente au moins par sections un matériau élastique.

4. Article pour le soin des plaies selon la revendication 1 à 3, dans lequel l'enveloppe est composée au moins partiellement d'une maille d'espacement de plaies ou est recouverte ou doublée par celle-ci.

5. Article pour le soin des plaies selon la revendication 1 à 4, dans lequel l'enveloppe est composée au moins partiellement d'un matériau imprégné ou imperméable à l'eau ou est recouverte ou doublée par celui-ci.

6. Article pour le soin des plaies selon l'une quelconque des revendications précédentes, présentant une fraction d'au moins un métal lourd élémentaire ou sous forme ionique.

7. Article pour le soin des plaies selon la revendication 6, dans lequel le métal lourd élémentaire ou sous forme ionique est choisi dans le groupe contenant le cuivre, le zinc et/ou l'argent.

8. Article pour le soin des plaies selon l'une quelconque des revendications précédentes, **caractérisé en ce que** la couche plane ou l'enveloppe est recouverte ou remplacée sur un côté par une feuille de protection.

9. Article pour le soin des plaies selon la revendication 8, **caractérisé en ce que** la feuille de protection s'étend au-delà d'une périphérie de l'article pour le soin des plaies et peut être appliquée sur la peau environnante de la plaie.

10. Article pour le soin des plaies selon l'une quelconque des revendications précédentes, **caractérisé en ce que** la couche plane ou l'enveloppe présente sur au moins un côté un revêtement adhésif.

11. Article pour le soin des plaies selon l'une quelconque des revendications précédentes, présentant en outre au moins un élément de fixation pour la fixation de celui-ci à une partie du corps.

12. Article pour le soin des plaies selon l'une quelconque des revendications précédentes, présentant en outre une ou plusieurs lignes de pliage, lignes de rupture ou rainures.

13. Article pour le soin des plaies selon l'une quelconque des revendications précédentes, pour l'utilisation dans un procédé de traitement de plaies par
• application sur une cuisse ou une jambe
• application dans la zone de l'épaule, de la poitrine ou du cou
• application dans la zone de l'aisselle
• application dans la zone inférieure ou supérieure de la poitrine
• application dans la zone des côtes
• application dans la zone de l'avant-bras ou du bras
• application dans la zone sous-oculaire
• application dans la zone sacrale
• application sur le dos
• application dans le creux du genou
• application dans la zone du bas-ventre
• application dans la zone arrière ou supérieure de l'oreille
• application dans la zone interdigitale et/ou
• application dans la zone du pied
d'un patient.

14. Article pour le soin des plaies selon l'une quelconque des revendications précédentes, pour l'utilisation dans un procédé de traitement de plaies par utilisation dans un système de soins des plaies à dépression.
